# EUROPEAN PATENT APPLICATION

(11) **EP 4 806 987 A1**
(43) Date of publication of application: **16.09.2026**
(21) Application number: 25874626.2
(22) Date of filing: 09.09.2025
(51) Int. Cl.: C12N 5/0735, C12N 5/074, C12N 5/0775

(54) **INDUCED DIFFERENTIATION CULTURE MEDIUM FOR MESENCHYMAL STEM CELLS AND USE THEREOF**

(30) Priority: 26.12.2024 CN 202411932891
(71) Applicant: Hunan Meibo Biomedical Co., Ltd, Changsha, Hunan 410300 (CN)
(72) Inventor: XIAO, E, Changsha, Hunan 410300 (CN); LIU, Qiushi, Changsha, Hunan 410300 (CN)
(74) Representative: Dai, Simin
(86) International application number: PCT/CN2025/119998
(87) International publication number: WO 2026/137983

(57) **Abstract**

The present disclosure belongs to the field of stem cell biology, relates to lineage-specific differentiation of human pluripotent or embryonic stem cells, and specifically relates to a induction/differentiation medium for producing of mesenchymal stem cells and a use thereof. The induction/differentiation medium is used for directly inducing differentiation of human induced pluripotent stem cells or embryonic stem cells into mesenchymal stem cells. The induction/differentiation medium includes a mesenchymal stem cell basal medium and additives. The additives include: 2 to 10 µM of a TGF-β inhibitor, 2 to 10 µM of a GSK3β inhibitor, and 5 to 10 µM of a ROCK inhibitor. The induction/differentiation medium provided in present disclosure can be used to directly induce human induced pluripotent stem cells or embryonic stem cells to differentiate into mesenchymal stem cells. The obtained mesenchymal stem cells offer advantages such as an unlimited source, stable quality, and high homogeneity. Additionally, this method allows for the directed differentiation of human induced pluripotent stem cells or embryonic stem cells into mesenchymal stem cells within a relatively short period, thereby reducing cultivation time and lowering production costs.

## Description

### TECHNICAL FIELD

The present disclosure generally relates to the field of stem cell biology, and in particular to lineage-specific differentiation of human induced pluripotent stem cells or embryonic stem cells, and specifically relates to a induction/differentiation medium for producing of mesenchymal stem cells and a preparation method thereof.

### BACKGROUND

Mesenchymal stem cells (MSCs) can be isolated from various human tissues, such as bone marrow, adipose tissue, umbilical cord blood, peripheral blood, neonatal tissue umbilical cord, placenta, or the like. However, a count of MSCs obtainable from adult tissues is limited, and an invasive procedure is required to isolate the MSCs, which also brings unexpected risks to a donor. Therefore, obtaining MSCs in sufficient quantity, with uniformity and stable quality, is key to realizing uses of the MSCs.

Human induced pluripotent stem cells include human embryonic stem cells (hESCs) and human induced pluripotent stem cells (iPSCs). The iPSCs can proliferate indefinitely in vitro while maintaining a potential to differentiate into almost all adult cell types. Directed differentiation of the iPSCs to obtain induced mesenchymal stem cells (iMSCs) provides a stable source of seed cells for MSC-based cell therapy. At present, numerous studies have reported acquisition of the MSCs using this strategy. Compared with tissue-derived MSCs, the iMSCs exhibit advantages such as higher homogeneity, more stable biological properties, and more predictable biological functions. However, MSCs are rare and exhibit heterogeneity among different tissues and organs, and the acquisition generally requires invasive procedures (except for those derived from umbilical cord), which limits potential of the MSCs for clinical translation. In addition, the MSCs have limited expansion capacity during culture and typically begin to undergo senescence after approximately 8 to 10 passages, making the MSCs difficult to generate a sufficient quantity of cells.

Therefore, a simple, reliable, and sufficient cell source is crucial for the translational application of the MSCs. The iPSCs are pluripotent stem cells obtained by inducing the adult cells through reprogramming technology, and possess characteristics similar to embryonic stem cells. iPSCs can be cultured and expanded in vitro without limitation, thereby continuously generating a large quantity of adult stem cells for use. Therefore, the iPSCs have gradually become an optimal cell source for obtaining the MSCs. At present, most established manners for differentiating the iPSCs into various cell types, including the MSCs, are based on spontaneous differentiation of embryoid bodies (EBs). Such manners usually require 30 to 40 days, with long processing time, low efficiency, and poor controllability. Another manner involves first inducing pluripotent stem cells to form trophoblast stem cells and subsequently differentiating the trophoblast stem cells into the MSCs. Such manners have problems such as incomplete detachment of upper-layer cells, long processing time, presence of mixed cell populations, insufficient differentiation, and low purity of the MSCs. Meanwhile, most existing disclosed manners for preparing the MSCs through directed induction of the iPSCs require adherent culture, enzymatic digestion, repeated passaging, and processes such as mouse-derived cell co-culture, coating with xenogeneic materials, flow cytometric sorting, or viral transfection. Such manners involve complicated and cumbersome operations, produce low yields with unstable quality, and are not suitable for large-scale production. Moreover, the introduction of exogenous xenogeneic substances during the culture process further limits practical applicability of the iPSCs.

Based on this, the present disclosure aims to provide a medium for induction and differentiation of the MSCs and a use thereof.

### SUMMARY

The present disclosure aims to provide a medium for induction and differentiation of the MSCs and a use thereof.

A first aspect of the present disclosure provides a induction/differentiation medium for producing of mesenchymal stem cells. The induction/differentiation medium is used for directly inducing differentiation of human induced pluripotent stem cells or embryonic stem cells into mesenchymal stem cells. The induction/differentiation medium includes a mesenchymal stem cell basal medium and additives. The additives include: 2 to 10 µM of transforming growth factor-β (TGF-β) inhibitor, 2 to 10 µM of glycogen synthase kinase-3β (GSK3β) inhibitor, and 5 to 10 µM of rho-associated coiled-coil containing protein kinase (ROCK) inhibitor.

Specifically, in the induction/differentiation medium, a dosage of the TGF-β inhibitor may be 2 µM, 3 µM, 4 µM, 5 µM, 6 µM, 7 µM, 8 µM, 9 µM, 10 µM, or a range formed by any two of the above values. The dosage is not limited to the listed values, and other unlisted values within the range are also applicable. Specifically, in the induction/differentiation medium, a dosage of the GSK3β inhibitor may be 2 µM, 3 µM, 4 µM, 5 µM, 6 µM, 7 µM, 8 µM, 9 µM, 10 µM, or a range formed by any two of the above values. The dosage is not limited to the listed values, and other unlisted values within the range are also applicable. Specifically, in the induction/differentiation medium, a dosage of the ROCK inhibitor may be 2 µM, 3 µM, 4 µM, 5 µM, 6 µM, 7 µM, 8 µM, 9 µM, 10 µM, or a range formed by any two of the above values. The dosage is not limited to the listed values, and other unlisted values within the range are also applicable.

In some embodiments of the present disclosure, the additives further include: human platelet lysate, ascorbic acid, GlutaMax additive, non-essential amino acids (NEAA), insulin-transferrin-selenium-ethanolamine (ITS-X) supplement, insulin-like growth factor (IGF), basic fibroblast growth factor (bFGF), and platelet-derived growth factor-BB (PDGF-BB).

In some embodiments of the present disclosure, the additives further include: human platelet lysate at a volume concentration of 1 to 10%; ascorbic acid at a concentration of 30 to 70 mM; GlutaMax additive at a volume concentration of 0.5% to 2%; NEAA at a volume concentration of 0.5% to 2%; ITS-X supplement at a volume concentration of 0.5% to 2%; IGF at a concentration of 1 to 10 ng/mL; bFGF at a concentration of 1 to 10 ng/mL; and PDGF-BB at a concentration of 1 to 10 ng/mL.

Specifically, in the induction/differentiation medium, a volume concentration of the human platelet lysate may be 1%, 2%, 3%, 4%, 5%, 6%, 7%, 8%, 9%, 10%, or a range formed by any two of the above values, and is not limited to the listed values, and other unlisted values within the range are also applicable. Specifically, in the induction/differentiation medium, a dosage of the ascorbic acid may be 30 mM, 31 mM, 32 mM, 33 mM, 34 mM, 35 mM, 36 mM, 37 mM, 38 mM, 39 mM, 40 mM, 41 mM, 42 mM, 43 mM, 44 mM, 45 mM, 46 mM, 47 mM, 48 mM, 49 mM, 50 mM, 51 mM, 52 mM, 53 mM, 54 mM, 55 mM, 56 mM, 57 mM, 58 mM, 59 mM, 60 mM, 61 mM, 62 mM, 63 mM, 64 mM, 65 mM, 66 mM, 67 mM, 68 mM, 69 mM, 70 mM, or a range formed by any two of the above values, and is not limited to the listed values, and other unlisted values within the range are also applicable. Specifically, in the induction/differentiation medium, a volume concentration of the GlutaMax additive may be 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2%, or a range formed by any two of the above values, and is not limited to the listed values, and other unlisted values within the range are also applicable. Specifically, in the induction/differentiation medium, a volume concentration of the NEAA may be 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2%, or a range formed by any two of the above values, and is not limited to the listed values, and other unlisted values within the range are also applicable. Specifically, in the induction/differentiation medium, a volume concentration of the ITS-X supplement may be 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2%, or a range formed by any two of the above values, and is not limited to the listed values, and other unlisted values within the range are also applicable. Specifically, in the induction/differentiation medium, a dosage of the IGF may be 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, or a range formed by any two of the above values, and is not limited to the listed values, and other unlisted values within the range are also applicable. Specifically, in the induction/differentiation medium, a dosage of the bFGF may be 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, or a range formed by any two of the above values, and is not limited to the listed values, and other unlisted values within the range are also applicable. Specifically, in the induction/differentiation medium, a dosage of the PDGF-BB may be 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, or a range formed by any two of the above values, and is not limited to the listed values, and other unlisted values within the range are also applicable.

In some embodiments of the present disclosure, the TGF-β inhibitor includes at least one of SB431542, A-8301, or LY2157299.

In some embodiments of the present disclosure, the GSK3β inhibitor includes at least one of CHIR99021, SB415286, or LY2090314.

In some embodiments of the present disclosure, the ROCK inhibitor includes at least one of Y-27632 or Blebbistatin.

In some embodiments of the present disclosure, the mesenchymal stem cell basal medium is a serum-free basal medium for mesenchymal stem cells; and the serum-free basal medium for mesenchymal stem cells includes at least one of high-glucose Dulbecco's Modified Eagle Medium (DMEM), alpha minimum essential medium (Alpha-MEM), or dulbecco's modified eagle medium/ham's f-12 nutrient mixture (DMEM/F12) medium.

A second aspect of the present disclosure further provides a method for directly inducing and differentiating human induced pluripotent stem cells or embryonic stem cells into mesenchymal stem cells. The method includes subjecting the human induced pluripotent stem cells or the embryonic stem cells to passaging culture in the induction/differentiation medium for producing of mesenchymal stem cells according to the first aspect of the present disclosure, thereby directly inducing and differentiating the human induced pluripotent stem cells or the embryonic stem cells into the mesenchymal stem cells.

In some embodiments of the present disclosure, the method includes:
step I. culturing a cell culture of the human induced pluripotent stem cells or the embryonic stem cells; and
step II. replacing a medium in the cell culture with the induction/differentiation medium to perform passaging culture, thereby obtaining P3~P5 passage mesenchymal stem cells or a cell culture including the P3~P5 passage mesenchymal stem cells.

In some embodiments of the present disclosure, in step I, the culturing the cell culture includes: culturing the human induced pluripotent stem cells or the embryonic stem cells in a xeno-free medium in a feeder-free culture manner; preferably, the step of culturing the cell culture further includes: first culturing the human induced pluripotent stem cells or the embryonic stem cells normally in an induced pluripotent stem cell (iPSC) maintenance medium; when confluence of the cell culture reaches 80% to 90%, digesting the human induced pluripotent stem cells with Tryple into a single-cell suspension; resuspending the single-cell suspension in an iPSC maintenance medium; adding the ROCK inhibitor to the iPSC maintenance medium; after maintaining the ROCK inhibitor for 24 h, and replacing with a complete iPSC maintenance medium to obtain the cell culture at 30% to 50% confluence;

In some embodiments of the present disclosure, the iPSC maintenance medium includes at least one of Chemically Defined Essential 8 Medium (E8), StemFit Basic04, or mTeSR Plus.

In some embodiments of the present disclosure, the ROCK inhibitor includes at least one of Y-27632 or Blebbistatin.

In some embodiments of the present disclosure, a matrix gel used in the culturing the cell culture includes at least one of Laminin-521, Vitronectin, or Matrigel.

In some embodiments of the present disclosure, culturing conditions for the step of culturing the cell culture include: culturing in an incubator at 35-40°C, 4-6% CO₂, and 92-98% humidity.

In some embodiments of the present disclosure, in step II, replacing the medium in the cell culture with the induction/differentiation medium to perform passaging culture includes: replacing the medium in the cell culture with the induction/differentiation medium; when confluence reaches 80%-90%, designating the cell culture as P0 passage mesenchymal stem cells; performing a digestion treatment on the P0 passage mesenchymal stem cells; resuspending the P0 passage mesenchymal stem cells after the digestion treatment using the induction/differentiation medium; continuing culturing to obtain P1 passage mesenchymal stem cells; and repeating the above passage culturing steps to obtain the P3~P5 passage mesenchymal stem cells.

In some embodiments of the present disclosure, the digestion treatment includes: aspirating supernatant; adding Ca²⁺ and Mg²⁺-free dulbecco's phosphate buffered saline (DPBS) to wash the P0 passage mesenchymal stem cells; performing the digestion treatment on the P0 passage mesenchymal stem cells after washing; centrifuging to remove supernatant; and obtaining the P0 passage mesenchymal stem cells after the digestion treatment.

In some embodiments of the present disclosure, the method includes: placing the P3~P5 passage mesenchymal stem cells or the cell culture including the P3~P5 passage mesenchymal stem cells in an expansion medium for mesenchymal stem cells to perform expansion passaging culture, thereby obtaining the mesenchymal stem cells.

In some embodiments of the present disclosure, the expansion medium for mesenchymal stem cells includes: a mesenchymal stem cell basal medium and additives; wherein the additives include: human platelet lysate, ascorbic acid, GlutaMax additive, NEAA, ITS-X supplement, IGF, bFGF, and PDGF-BB;

In some embodiments of the present disclosure, the additives further include: human platelet lysate at a volume concentration of 1 to 10%; ascorbic acid at a concentration of 30 to 70 mM; GlutaMax additive at a volume concentration of 0.5% to 2%; NEAA at a volume concentration of 0.5% to 2%; ITS-X supplement at a volume concentration of 0.5% to 2%; IGF at a concentration of 1 to 10 ng/mL; bFGF at a concentration of 1 to 10 ng/mL; and PDGF-BB at a concentration of 1 to 10 ng/mL.

Specifically, in the induction/differentiation medium, a volume concentration of the human platelet lysate may be 1%, 2%, 3%, 4%, 5%, 6%, 7%, 8%, 9%, 10%, or a range formed by any two of the above values, and is not limited to the listed values, and other unlisted values within the range are also applicable. Specifically, in the induction/differentiation medium, a dosage of the ascorbic acid may be 30 mM, 31 mM, 32 mM, 33 mM, 34 mM, 35 mM, 36 mM, 37 mM, 38 mM, 39 mM, 40 mM, 41 mM, 42 mM, 43 mM, 44 mM, 45 mM, 46 mM, 47 mM, 48 mM, 49 mM, 50 mM, 51 mM, 52 mM, 53 mM, 54 mM, 55 mM, 56 mM, 57 mM, 58 mM, 59 mM, 60 mM, 61 mM, 62 mM, 63 mM, 64 mM, 65 mM, 66 mM, 67 mM, 68 mM, 69 mM, 70 mM, or a range formed by any two of the above values, and is not limited to the listed values, and other unlisted values within the range are also applicable. Specifically, in the induction/differentiation medium, a volume concentration of the GlutaMax additive may be 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2%, or a range formed by any two of the above values, and is not limited to the listed values, and other unlisted values within the range are also applicable. Specifically, in the induction/differentiation medium, a volume concentration of the NEAA may be 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2%, or a range formed by any two of the above values, and is not limited to the listed values, and other unlisted values within the range are also applicable. Specifically, in the induction/differentiation medium, a volume concentration of the ITS-X supplement may be 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2%, or a range formed by any two of the above values, and is not limited to the listed values, and other unlisted values within the range are also applicable. Specifically, in the induction/differentiation medium, a dosage of the IGF may be 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, or a range formed by any two of the above values, and is not limited to the listed values, and other unlisted values within the range are also applicable. Specifically, in the induction/differentiation medium, a dosage of the bFGF may be 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, or a range formed by any two of the above values, and is not limited to the listed values, and other unlisted values within the range are also applicable. Specifically, in the induction/differentiation medium, a dosage of the PDGF-BB may be 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, or a range formed by any two of the above values, and is not limited to the listed values, and other unlisted values within the range are also applicable.

In some embodiments of the present disclosure, the mesenchymal stem cell basal medium is a serum-free basal medium for mesenchymal stem cells; and the serum-free basal medium for mesenchymal stem cells includes at least one of high-glucose DMEM, Alpha-MEM, or DMEM/F12 medium.

A third aspect of the present disclosure further provides mesenchymal stem cells prepared by the method for directly inducing and differentiating human induced pluripotent stem cells or embryonic stem cells into mesenchymal stem cells provided according to the second aspect of the present disclosure.

In some embodiments of the present disclosure, the mesenchymal stem cell is a multipotent cell capable of differentiating into an adipocyte, an osteocyte, a chondrocyte, a myocyte, a neuron, and a cardiomyocyte.

In some embodiments of the present disclosure, the mesenchymal stem cells are capable of expressing cell surface markers Cluster of Differentiation (CD) 29, CD73, CD90, CD105, CD166, and CD44.

In some embodiments of the present disclosure, the mesenchymal stem cells do not express cell surface markers Human Leukocyte Antigen-DR isotype (HLA-DR), CD34, CD45, CD19, and CD14.

In some embodiments of the present disclosure, the mesenchymal stem cells are P5 or higher passage mesenchymal stem cells; in the mesenchymal stem cells, a proportion of cells expressing CD29, CD44, CD73, CD90, CD105, and CD166 is not less than 95%; and in the mesenchymal stem cells, a proportion of cells expressing HLA-DR, CD34, CD45, CD19, and CD14 is not more than 2%.

Compared with the prior art, the present disclosure has the following beneficial effects:
(1) The present disclosure provides a medium for directly inducing and differentiating human induced pluripotent stem cells into mesenchymal stem cells and a method for directly inducing and differentiating human induced pluripotent stem cells into mesenchymal stem cells. By using the induction/differentiation medium for producing of mesenchymal stem cells provided by the present disclosure, human induced pluripotent stem cells can be directly induced to differentiate into mesenchymal stem cells. A cell differentiation pathway is clear, a differentiation efficiency is high, and a differentiation effect is stable. A culture system containing serum or feeder cells is not used. The obtained cell population has high purity and large quantity, thereby addressing the problems in the prior art, such as long induction and differentiation time, long passaging interval, and slow cell proliferation. The serum-free and xeno-free culture system solves safety problems. Moreover, even after repeated passaging culture, characteristics of the mesenchymal stem cells can be maintained over a long period.
(2) The present disclosure significantly improves a differentiation efficiency of the mesenchymal stem cells, and even after long-term passaging culture (for example, 12 passages, or even 15 passages or more), the obtained cells still exhibit excellent performance in stably maintaining characteristics of the mesenchymal stem cells. Through such passaging, mesenchymal stem cells derived from the human induced pluripotent stem cells can be prepared in large quantities. During the differentiation process, differentiation pathways are precisely controlled through combined use of small molecule compounds, achieving stable and efficient differentiation, and finally obtaining mature MSCs. The obtained MSCs express cell surface markers Cluster of Differentiation (CD) 90, CD73, CD105, and do not express CD14, CD34, CD45, CD19, and Human Leukocyte Antigen-DR isotype (HLA-DR). The MSCs grow in an adherent manner and possess osteogenic and adipogenic differentiation potential, without requiring additional flow cytometric sorting for cell selection.
(3) Compared with an induction manner disclosed in the prior art where pluripotent stem cells are first induced to trophoblast stem cells and the trophoblast stem cells are subsequently differentiated into mesenchymal stem cells, such approaches suffer from problems including incomplete detachment of upper-layer cells, long processing time, the presence of mixed cell populations, insufficient differentiation, and low purity of mesenchymal stem cells. By using the induction/differentiation medium for producing of mesenchymal stem cells provided in the present disclosure, the human induced pluripotent stem cells can be directly induced to differentiate into the mesenchymal stem cells. The process described herein requires a shorter time and establishes an induction and differentiation system with clearly defined components. A serum-free culture system enables directed induction and differentiation to efficiently obtain mesenchymal stem cells with uniform purity and stable performance. Compared with the spontaneous differentiation of EBs disclosed in the prior art, which has problems such as low induction efficiency, possible retention of other pluripotent cells, long processing time, labor-intensive procedures, low efficiency, and poor controllability, the induction and differentiation medium provided herein enables direct differentiation of the human induced pluripotent stem cells into the mesenchymal stem cells, thereby simplifying the experimental procedure and providing good reproducibility. That is, compared with prior art manners involving spontaneous differentiation through EB formation or induction in which the pluripotent stem cells are first converted into trophoblast stem cells and then differentiated into the mesenchymal stem cells, the method provided in the present disclosure requires a shorter time and uses a defined-component serum-free induction and differentiation system to achieve efficient directed monolayer differentiation, thereby obtaining mesenchymal stem cells with uniform purity and stable performance.

### BRIEF DESCRIPTION OF THE DRAWINGS

FIG. 1 is a schematic diagram illustrating pluripotency identification results of human induced pluripotent stem cell according to Example 1 of the present disclosure.
FIG. 2 is a schematic diagram illustrating a morphology of iPSC-derived iMSCs at different passages obtained according to Example 2 of the present disclosure.
FIG. 3A is a schematic diagram illustrating proportions of CD90+, CD73+, CD105+, and CD45- in iMSCs detected by flow cytometry according to some effect examples of the present disclosure.
FIG. 3B is a schematic diagram illustrating proportions of CD14-, CD34-, CD19-, and HLA-DR- phenotypes in iMSCs detected by flow cytometry according to some effect examples of the present disclosure.
FIG. 4 is a schematic diagram illustrating detection results of expression of MSC-related marker genes *OCT4*, *SNAI2*, *COL6A2*, and *TWIST1* by RT-qPCR according to some effect example of the present disclosure.
FIG. 5 is a schematic diagram illustrating detection results of expression of osteogenic-specific genes *OCN*, *ALP*, and *RUNX2* in iMSCs according to some effect examples of the present disclosure.
FIG. 6 is a schematic diagram illustrating detection results of osteogenic differentiation capability of iMSCs according to some effect examples of the present disclosure.
FIG. 7 is a schematic diagram illustrating detection results of expression of adipogenic-specific genes *PPARγ2* and *LPL* in iMSCs according to some effect examples of the present disclosure.
FIG. 8 is a schematic diagram illustrating detection results of adipogenic differentiation capability of iMSCs according to some effect examples of the present disclosure.
FIG. 9 is a schematic diagram illustrating cell culture states of No. 1 to No. 6 different induction and differentiation media in comparative examples of the present disclosure.
FIG. 10 is a schematic diagram illustrating cell states on day 6 of differentiation using iPSC-iMSC induction and differentiation media of Group A, Group B, and Group C according to comparative examples of the present disclosure.

### DETAILED DESCRIPTION

The technical solutions of the present disclosure are further illustrated below by specific examples. The specific examples do not represent a limitation on the scope of protection of the present disclosure. Some non-essential modifications and adjustments made by others based on the concept of the present disclosure still fall within the scope of protection of the present disclosure.

Information on cells and reagents used in the examples of the present disclosure is as follows:
Human induced pluripotent stem cells: Catalog number: hCiPSC-00409, Manufacturer: Beijing Beigi Biomedicine Co., Ltd.;
Laminin-521 stock solution: Catalog number: 200-0117, Manufacturer: STEMCELL Technologies Inc.;
DPBS (with calcium and magnesium): Catalog number: 14080055, Manufacturer: Gibco, Thermo Fisher Scientific Inc.;
DPBS (without calcium and magnesium): Catalog number: C14190500BT, Manufacturer: Gibco, Thermo Fisher Scientific Inc.;
Vitronectin stock solution: Catalog number: RP01002, Manufacturer: Sino Biological Inc.;
DMEM/F12: Catalog number: 11330-032, Manufacturer: Gibco, Thermo Fisher Scientific Inc.;
mTeSR Plus medium: Catalog number: 100-0276, Manufacturer: STEMCELL Technologies Inc.;
TrypLE^{™} Express: Catalog number: 12604021, Manufacturer: Gibco, Thermo Fisher Scientific Inc.;
Y27632: Catalog number: HY-10071, Manufacturer: MedChemExpress LLC;
Versene digestion enzyme: Catalog number: 15040066, Manufacturer: Gibco, Thermo Fisher Scientific Inc.;
Human platelet lysate: Catalog number: PL-NH-100, Manufacturer: Sexton Biotechnologies, Inc.;
Ascorbic acid: Catalog number: A8960, Manufacturer: Merck KGaA;
GlutaMax additive: Catalog number: A1286001, Manufacturer: Gibco, Thermo Fisher Scientific Inc.;
NEAA: Catalog number: 11140050, Manufacturer: Gibco, Thermo Fisher Scientific Inc.;
ITS-X supplement: Catalog number: 51500056, Manufacturer: Gibco, Thermo Fisher Scientific Inc.;
IGF: Catalog number: GMP-C023, Manufacturer: ACROBiosystems Co., Ltd.;
bFGF: Catalog number: GMP-C046, Manufacturer: ACROBiosystems Co., Ltd.;
PDGF-BB: Catalog number: GMP-C199, Manufacturer: ACROBiosystems Co., Ltd..

### Example 1 Culture and passage of initial cells

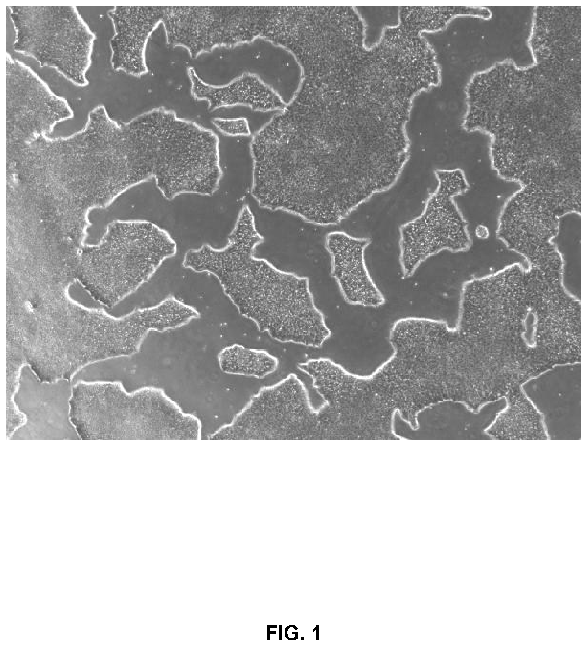

This example used iPSCs as initial cells. As specifically shown in FIG. 1, according to results shown in FIG. 1, the iPSCs exhibited typical colony growth characteristics, with clear colony edges, tight cell contacts within colonies, a high nuclear-to-cytoplasmic ratio, a uniform morphology, and no differentiated cells observed. Culture and passage operations for the iPSCs were as follows:

Coating a culture plate with Laminin-521: A Laminin-521 stock solution was taken, and the Laminin-521 was diluted to a final concentration of 5 µg/mL using DPBS (with calcium and magnesium). The diluted Laminin-521 was added to the culture plate, and the culture plate was placed in a refrigerator at 2-8°C overnight or at 37°C for at least 2 hours for coating. After the coating was completed, the culture plate was taken out of the refrigerator and placed at a room temperature for 30 minutes before use.

Coating a culture plate with Vitronectin: A Vitronectin stock solution was taken, and the Vitronectin stock solution was diluted to a final concentration of 10 µg/mL using DMEM/F12. The diluted Vitronectin was added to the culture plate, and the culture plate was placed in a refrigerator at 2-8°C overnight or at a room temperature for at least 1 hour for coating. After the coating was completed, the culture plate was taken out of the refrigerator and placed at a room temperature for 30 minutes before use.

Cell thawing: A thawed iPSC suspension was transferred to a new 15 mL centrifuge tube, and an iPSC complete medium equilibrated to a room temperature was gently added dropwise to the iPSC suspension while gently shaking the centrifuge tube. The 15 mL centrifuge tube containing the iPSC suspension was transferred to a low-speed refrigerated centrifuge and centrifuged at 300 g for 3 minutes at a room temperature. After centrifugation, supernatant was discarded, and an iPSC complete medium (mTeSR Plus) containing 10 µM Y27632 was added to resuspend the cells. The cells were gently pipetted and seeded at a volume ratio of 1:15 into a pre-warmed and prepared 12-well culture plate. The cells were cultured in an incubator at 37°C, 5% CO₂ concentration, and 95% humidity. The medium was changed every 20 to 24 hours. Cell passage was started when confluence of the cells reached more than 80%.

Cell passaging: When the confluence of the cells reached more than 80%, the cell culture plate was taken out of the incubator. The cells were washed once with DPBS (without calcium and magnesium). Then, a Versene digestion enzyme was added to the culture plate to digest the cells for 3 to 5 minutes. A state of colony dissociation was observed under a microscope. After the cells were completely dissociated, the iPSC complete medium (mTeSR Plus) was added and the cell suspension was gently pipetted to disperse the cells. Another blank culture plate pre-warmed to 37°C was taken out of the incubator. The cell suspension was added at a volume ratio of 1:20. The culture plate was placed in the incubator at 37°C and 5% CO₂ concentration for overnight culture. The medium was changed every 20 to 24 hours. A next round of the cell passaging was started when the confluence of the cells reached more than 80%. According to the above manner, the cells were passaged to P20 (Passage 20) to obtain P20 passage cells.

### Example 2

This example provides a preparation manner for direct induction and differentiation of iPSCs into MSCs, including:
Step I, human induced pluripotent stem cell culture: the iPSCs passaged to the P20 passage prepared in Example 1 were normally cultured in an iPSC maintenance medium. The iPSC maintenance medium used was mTeSR Plus. When the iPSCs were cultured to confluence of 80% to 90%, the iPSCs were digested into a complete single-cell suspension using Tryple. The cells were resuspended in the iPSC maintenance medium. A ROCK inhibitor was added to the iPSC maintenance medium. After the ROCK inhibitor was maintained for 24 hours, the medium was replaced with a complete iPSC maintenance medium. The iPSCs were cultured using a 12-well plate. The iPSC maintenance medium used was mTeSR Plus. The ROCK inhibitor was Y-27632. A concentration of the ROCK inhibitor was 10 µM. Confluence of the cultured iPSCs was 30% to 50%.
Step II. Induction and differentiation of iPSCs into MSCs:

The iPSC maintenance medium for iPSCs in step I was replaced with a induction/differentiation medium for producing of mesenchymal stem cells. The cells were placed in a constant temperature incubator at 5% CO₂ and 37°C for culture. The cells were designated as P0 at this time. When confluence reached 80% to 90%, supernatant was aspirated. Pre-warmed Ca²⁺ and Mg²⁺-free DPBS was added to wash the cells twice. Then, pre-warmed Tryple was added to digest the cells until the cells reached a single-cell state. Digestion was terminated. After centrifugation, the supernatant was removed. The cells were resuspended using 1 mL of the induction/differentiation medium for producing of mesenchymal stem cells. The cells were seeded at a density of 5×10⁴ cells/cm² into a well plate pre-coated with Laminin-521. The well plate was placed in a constant temperature incubator at 5% CO₂ and 37°C for culture. The cells were designated as P1 at this time. According to the above steps, the cells were passaged and cultured to P3 passage. A composition of the induction/differentiation medium for producing of mesenchymal stem cells was: a serum-free basal medium for mesenchymal stem cells (a basal medium was selected from DMEM/F12 medium), containing 5% (v/v) human platelet lysate, 50 mM ascorbic acid, 1% (v/v) GlutaMax additive, 1% (v/v) NEAA, 1% (v/v) ITS-X supplement, 5 ng/mL IGF, 5 ng/mL bFGF, 5 ng/mL PDGF-BB, 5 µM TGF-β inhibitor (A8301), 5 µM GSK3β inhibitor (CHIR99021), and 8 µM ROCK inhibitor (Y-27632).

### Step III. Expansion passaging culture of iMSCs

When confluence of the P3 passage cells obtained in step II reached about 80% to 90%, the cells were digested again to the single-cell state. The cells were resuspended in 1 mL of an expansion medium for mesenchymal stem cells. The cells were seeded at a density of 0.9×10⁴ cells/cm² onto a plate pre-coated with the Laminin-521. The plate was placed in a 5% CO₂, 37°C incubator for culture for 3-5 days. When the confluence of the cells reached about 80% to 90%, the cells were subjected to passaging culture in a same manner as described above. The cells were passaged to P12 (Passage 12). P3 passage MSCs to P12 passage MSCs were obtained. A composition of the expansion medium for mesenchymal stem cells was: a serum-free basal medium for mesenchymal stem cells (a basal medium was selected from a DMEM/F12 medium), containing 5% (v/v) human platelet lysate, 50 mM ascorbic acid, 1% (v/v) GlutaMax additive, 1% (v/v) NEAA, 1% (v/v) ITS-X supplement, 5 ng/mL of IGF, 5 ng/mL of bFGF, and 5 ng/mL of PDGF-BB.

### Effect example 1

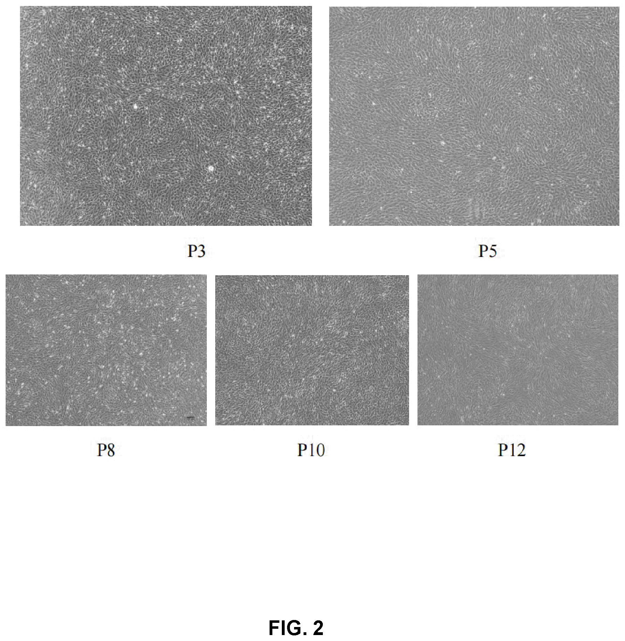

A morphology of the P3 passage and the subsequent passage MSCs prepared according to Example 2 was observed. Results are shown in FIG. 2. The P3 to P12 passage MSCs all exhibited a characteristic spindle-shaped morphology of MSCs arranged in parallel or spiral patterns. No signs of cellular senescence, such as an increased cell size or a reduced proliferation rate, were observed, and the cells were able to proliferate and be passaged stably.

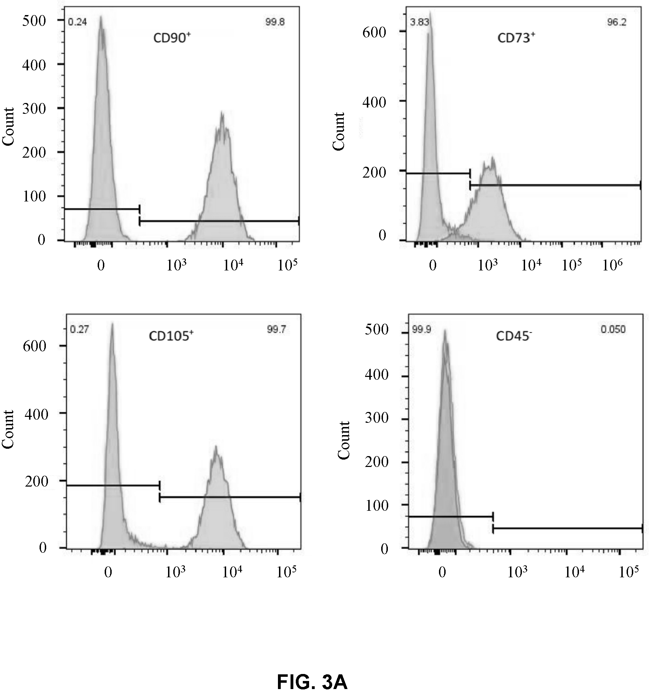

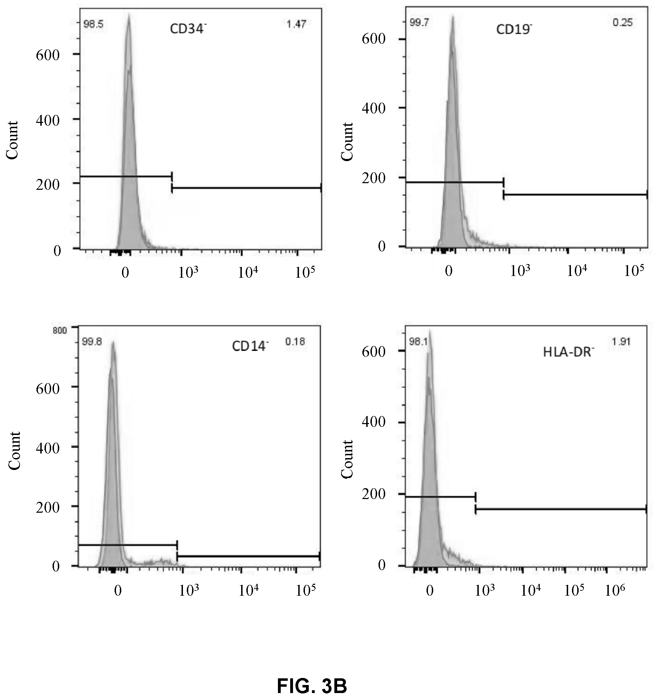

Meanwhile, P8 passage MSCs prepared in Example 2 were collected and analyzed by flow cytometry. Detection results are shown in FIG. 3. The MSCs exhibited expression levels greater than 95% for CD73, CD90, and CD105, indicating positive expression, while the expression levels of CD19, CD14, CD34, CD45, and HLA-DR were less than 2%, indicating negative expression. It should be specifically noted that, although the P8 passage cells were tested for flow cytometry analysis in this experimental experiment of the present disclosure, according to latest experimental data, the cells can be stably maintained for use up to passage P12.

### Effect example 2

To verify an effect of iPSC gradually differentiating into iMSC in the induction/differentiation medium for producing of mesenchymal stem cells provided by the present disclosure, this experimental example further verified the above effect by detecting expression of related genes in related cells. Specific steps were as follows:

The experiment was divided into 3 groups: an iMSC group (P1, P3, P5, P7 passage MSCs prepared in Example 2 of the present disclosure), an iPSC group (prepared in Example 1 of the present disclosure), and an ADSC group (adipose MSCs). RNA was extracted from the above 3 groups of cells, respectively. Expression of *OCT4, TWIST1, COL6A2,* and *SNAI2* was detected using a quantitative real-time polymerase chain reaction (qRT-PCR) manner. Specific steps were as follows:
Step I, RNA extraction: extraction was performed using an RNA purification kit (manufacturer: TransGen Biotech Co., Ltd., catalog number: ER101-01) to obtain a RNA sample.
Step II, reverse transcription: 1 µg of the RNA sample was taken. Reverse transcription was performed according to requirements of a chain reaction reverse transcription kit (manufacturer: TransGen Biotech Co., Ltd., catalog number: AU341-02). A reverse transcription system is as follows:

| Component | Volume |
|---|---|
| RNA | 1µg |
| 4× RT mix | 4µL |
| dd H₂O | Make up to 15 µL |

The above samples were mixed and centrifuged. The samples were incubated at 42°C for 15 min, and inactivated at 85°C for 5 s. The obtained cDNA was diluted 2 times for subsequent reactions.

Step III, quantitative Real-Time PCR (qRT-PCR): The samples obtained above were subjected to qRT-PCR detection according to the following system. A reaction system is as follows:

| Component | Volume |
|---|---|
| cDNA | 2µL |
| 2× SYBR Mix | 10µL |
| Forward primer | 0.5µL |
| Reverse primer | 0.5µL |
| dd H₂O | 7µL |

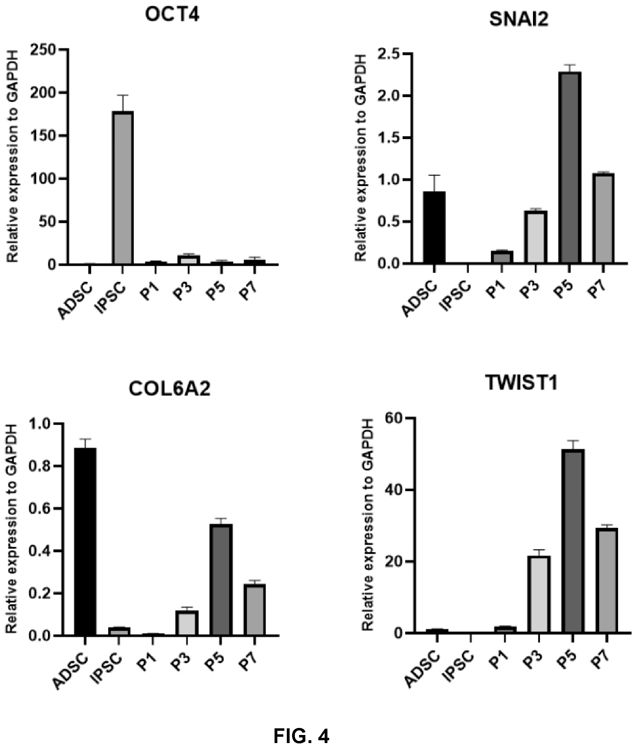

Detection results obtained through the above manner are shown in FIG. 4. According to the results shown in FIG. 4, MSCs of different passages exhibited significant expression of *TWIST1, COL6A2,* and *SNAI2.* According to gene expression results of P1, P3, P5, and P7 passage cells shown in FIG. 4, the cells had clearly undergone epithelial-to-mesenchymal transition (EMT). Combined with flow cytometry detection results of P8 passage cells shown in FIG. 3, it further indicates that the cells had differentiated into the iMSCs. *OCT4* is a marker gene of the iPSC. When iPSCs successfully differentiated into iMSCs, cells of each passage exhibited a situation of not expressing the *OCT4*. As shown in FIG. 4, P1, P3, P5, and P7 passage cells almost did not express the *OCT4* or expressed extremely trace amounts. This further indicates that, through the method for direct induction and differentiation provided by the present disclosure, the iPSCs can be successfully induced to differentiate into the iMSCs.

### Effect example 3

Osteogenic and adipogenic differentiation capabilities of P8 passage MSCs prepared in Example 2 were further detected as follows:

### Step I. Osteogenic differentiation identification:

P8 passage MSCs were seeded into a cell culture container at a suitable seeding density. An appropriate amount of a pre-warmed fresh iMSC expansion medium was added. The container was shaken horizontally in a cross pattern three times. The container was placed in a 37°C, 5% CO₂ concentration, saturated humidity incubator. The container was shaken horizontally in the cross pattern three times again before culturing. The iMSCs spread and grew uniformly. After the culturing, when confluence of the iMSCs reached 80%-90%, the medium in the container was aspirated and replaced with an osteogenic differentiation induction medium, which was recorded as day 0. The medium was completely replaced every three days. The culture was continued until day 21. A composition of the osteogenic differentiation induction medium used was: an Alpha-MEM, containing 10% FBS, 1% Penicillin-Streptomycin, 10 mM β-glycerophosphate, 10 nM Dexamethasone, and 50 µg/mL Ascorbic Acid (L-ascorbic acid).

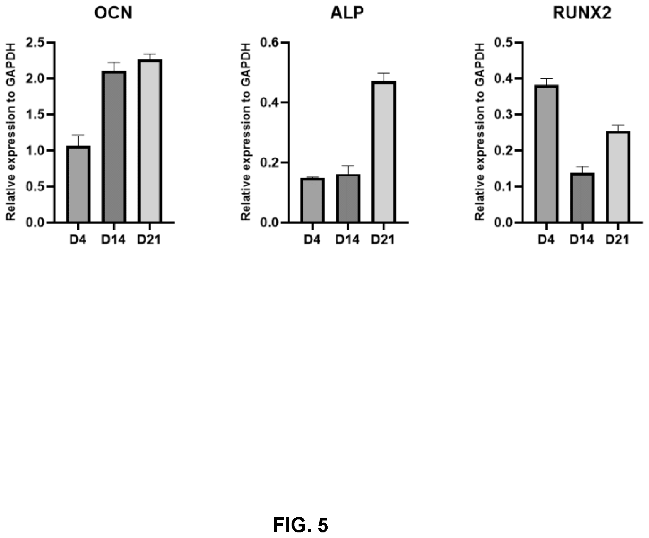

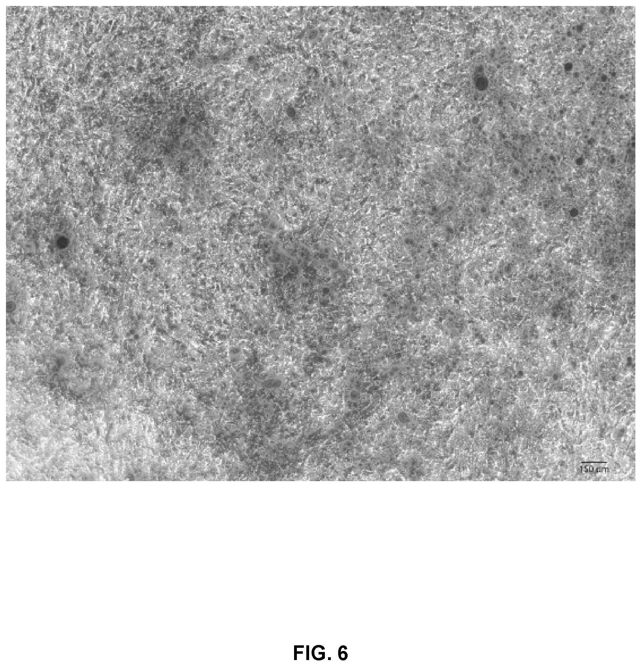

Differentiated cells cultured to day 4, day 14, and day 21 were observed and photographed under a light microscope. RNA of the differentiated cells was collected. Expression of osteogenic-specific genes *RUNX2*, *ALP*, and *OCN* was tested. Results are shown in FIG. 5. According to the results shown in FIG. 5, as a differentiation culture time increased, expression levels of specific genes *ALP* and *OCN* gradually increased. An expression level of a specific gene *RUNX2* first decreased and then increased. This indicates that the differentiated iMSCs had ability to differentiate into osteocytes. After day 21, the osteogenic-differentiated iMSCs were washed with pure water. An appropriate volume of Alizarin Red working solution was added. The iMSCs were incubated at a room temperature in dark for 20-30 min. Excess staining solution was then aspirated and discarded. An appropriate volume of saline or DPBS was added to each well to keep the cells hydrated. The iMSCs were observed under a microscope and photographed. Results are shown in FIG. 6. According to the results shown in FIG. 6, obvious calcium nodule formation was observed.

### Step II. Adipogenic differentiation identification:

P8 passage MSCs were seeded into a cell culture container at a suitable seeding density. An appropriate amount of the pre-warmed fresh iMSC expansion medium was added. The container was shaken horizontally in the cross pattern three times. The container was placed in a 37°C, 5% CO₂ concentration, saturated humidity incubator. The container was shaken horizontally in the cross pattern three times again before culturing. The iMSCs spread and grew uniformly. After the culturing, when confluence of the iMSCs reached 80%-90%, the medium in the container was aspirated and replaced with an adipogenic differentiation induction medium, which was recorded as day 0. The medium was completely replaced every three days. The culturing was continued until day 21. A composition of the adipogenic differentiation induction medium used was: an Alpha-MEM, containing 10% FBS, 1% Penicillin-Streptomycin, 1 µM Dexamethasone, 0.5 mM 3-isobutyl-1-methylxanthine (IBMX), 0.2 mM Indomethacin, and 10 µg/mL insulin.

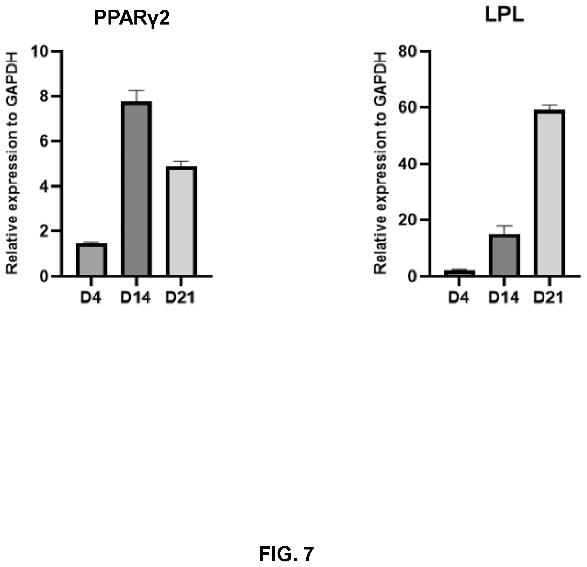

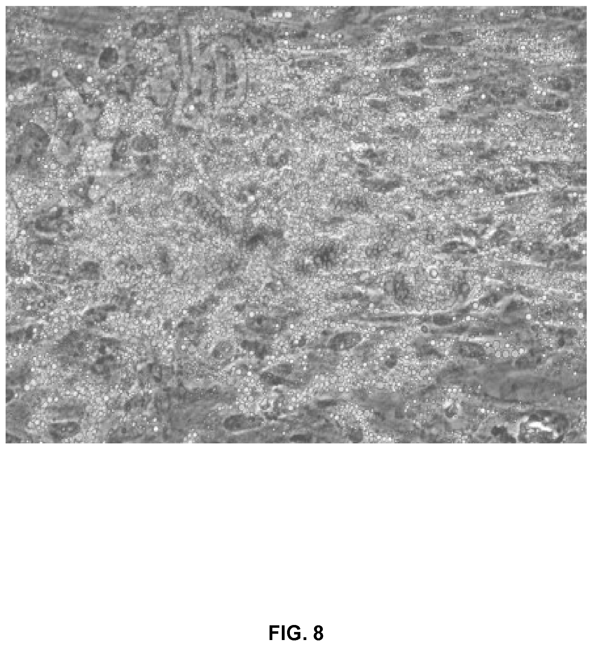

Differentiated cells cultured to day 4, day 14, and day 21 were observed and photographed under the light microscope. RNA of the differentiated cells was collected. Expression of adipogenic-specific genes *PPARγ2* and *LPL* was tested. Results are shown in FIG. 7. According to the results shown in FIG. 7, as a differentiation culture time increased, an expression level of *PPARγ2* first increased and then decreased. An expression level of *LPL* showed a trend of gradual increase. This indicates that the differentiated iMSCs had ability to differentiate into adipocytes. Simultaneously, during a normal differentiation process, it could be observed that the cells gradually became wider and shorter. Under high magnification, numerous round lipid droplets could be seen inside the cells. After day 21, the adipogenic-differentiated iMSCs were washed with the saline or the DPBS. The iMSCs were then washed once with 60% isopropanol solution to prevent residual saline or DPBS from causing precipitation of the staining solution. An appropriate volume of Oil Red O working solution was added to a differentiation group and a control group. The iMSCs were incubated at a room temperature in dark for 20-60 min. Excess staining solution was then aspirated and discarded. The iMSCs were washed with the saline or the DPBS until no background staining was observed. An appropriate volume of the saline or the DPBS was added to each well to keep the cells hydrated. The iMSCs were observed under a microscope and photographed. Results are shown in FIG. 8. According to the results shown in FIG. 8, obvious lipid droplet formation was observed.

### Comparative example

This comparative example further studied effects of different induction and differentiation media used for culturing iPSCs on related performance of finally obtained MSCs. Details are as follows:

The following groups were set respectively:
Medium No.1: an iMSC expansion medium.
Medium No. 2: 5 µM of a GSK3β inhibitor (CHIR99021) was added to an iMSC expansion medium;
Medium No. 3: 5 µM of a TGF-β inhibitor (A8301) was added to an iMSC expansion medium;
Medium No. 4: 8 µM of a ROCK inhibitor (Y-27632) was added to an iMSC expansion medium;
Medium No. 5: 5 µM of a GSK3β inhibitor (CHIR99021) and 5 µM of a TGF-β inhibitor (A8301) were added to an iMSC expansion medium;
Medium No. 6: 5 µM of a GSK3β inhibitor (CHIR99021), 5 µM of a TGF-β inhibitor (A8301), and 8 µM of a ROCK inhibitor (Y-27632) were added to an iMSC expansion medium;

The iMSC expansion medium has a composition including: a serum-free basal medium for mesenchymal stem cells (the basal medium is selected from a DMEM/F12 medium), 5% (v/v) human platelet lysate, 50 mM ascorbic acid, 1% (v/v) GlutaMax additive, 1% (v/v) NEAA, 1% (v/v) ITS-X supplement, 5 ng/mL of IGF, 5 ng/mL of bFGF, and 5 ng/mL of PDGF-BB;

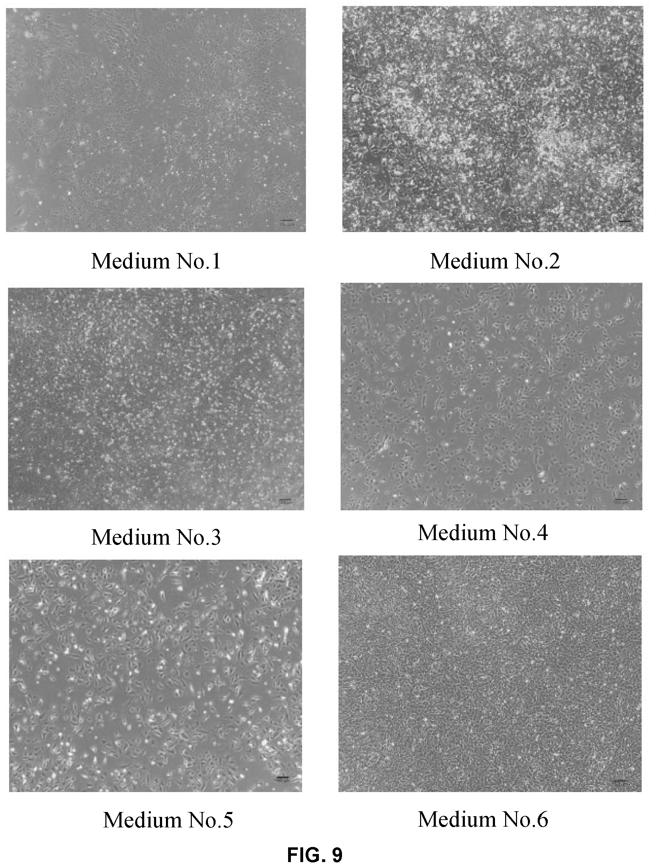

After passaging the iPSC prepared in Example 1, the old medium was removed. The iPSCs were divided into six groups. The six groups were respectively replaced with the six media described above. The cells were cultured in an incubator at 5% CO₂ and 37°C. A corresponding fresh medium for induction and differentiation was replaced daily. A cell morphology and proliferation were observed, and the cells were passaged when confluence of the cells reached 85% to 90%. During a differentiation process from the iPSCs to the iMSCs, the cell morphology was observed. Results are shown in FIG. 9. According to the results shown in FIG. 9, cells cultured in the Medium No. 1 exhibited almost no change in morphology and showed little proliferation. Cells cultured in the medium No. 2 gradually died during differentiation. Cells cultured in the medium No. 3 also showed almost no morphological changes. Cells cultured in the media No. 4 and the No. 5 showed no obvious proliferation when passaged to P1. Cells cultured in the medium No. 6 could be passaged to P2, and normal adherence was observed, with most cells exhibiting a short spindle-shaped morphology.

A further comparison was conducted to evaluate the effects of the following three groups of media for induction and differentiation on a differentiation of the iPSCs and a preparation of the MSCs. Details are as follows:
Group A medium: the Medium No. 6;
Group B medium: a basal medium E6 (brand: Gibco, catalog number: A1516401), added with 10 µM of a TGF-β inhibitor (SB431542), 2 µM of a GSK3β inhibitor (CHIR99021), and 10 ng/mL of bFGF;
Group C medium: a basal medium E6 (brand: Gibco, catalog number: A1516401), added with 0.1 mM of 2-mercaptoethanol, 1% of ITS-X supplement, 1.5 µg/mL of L-AA-pi, 50 ng/mL of Epidermal Growth Factor (EGF), 2 µM of a GSK3β inhibitor (CHIR99021), 0.5 µM of a TGF-β inhibitor (A83-01), 1 µM of a TGF-β inhibitor (SB431542), 0.8 mM of Valproic Acid (VPA), 5 µM of a ROCK inhibitor (Y27632), and 10 ng/mL of Bone Morphogenetic Protein 4 (BMP4).

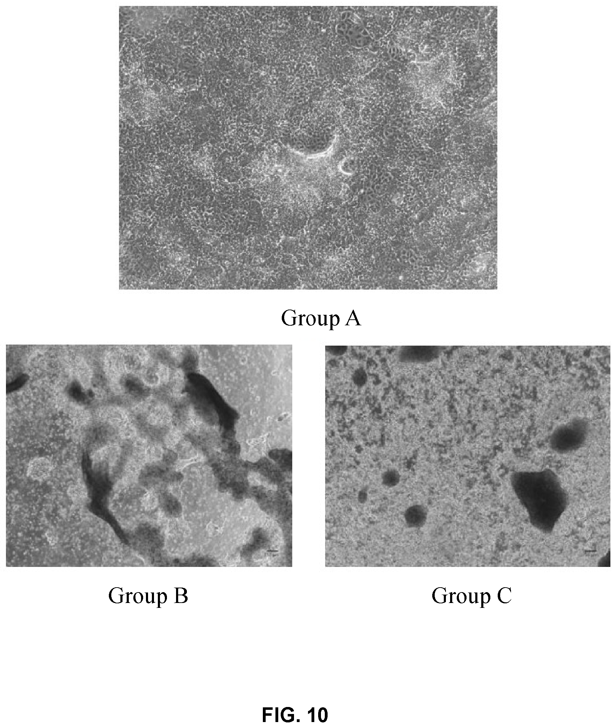

Single-cell iPSCs prepared in Example 1 were seeded and cultured for 2 days. When confluence reached about 30%, the iPSCs were divided into three groups. The three groups were respectively replaced with iPSC-iMSC induction and differentiation media of Group A, Group B, and Group C. A corresponding fresh medium was replaced daily. On day 6 of culture, a corresponding cell morphology was observed. Results are shown in FIG. 10. According to the results shown in FIG. 10, cells cultured in medium No. 6 of the group A exhibited a good differentiation state. Mesenchymal-like cells were observed under a microscope. In contrast, varying degrees of cell death occurred in the Group B and the Group C by day 6 of culture.

Combined with the results of the cell morphology and proliferation shown in FIG. 9 and FIG. 10, it further indicated that direct differentiation culture of the iPSCs using the induction/differentiation medium for producing of mesenchymal stem cells provided in the present disclosure can yield MSCs with excellent overall performance.

It is to be understood that the present disclosure is described by way of some embodiments. As is known to those skilled in the art, various changes or equivalent replacements may be made to these features and embodiments without departing from the spirit and scope of the present disclosure. In addition, under the teaching of the present disclosure, these features and embodiments may be modified to adapt to specific situations and materials without departing from the spirit and scope of the present disclosure. Therefore, the present disclosure is not limited by the specific embodiments disclosed herein. All embodiments falling within the scope of the claims of the present disclosure belong to the protection scope of the present disclosure.

## Claims

1. A induction/differentiation medium for producing of mesenchymal stem cells, wherein the induction/differentiation medium is used for directly inducing differentiation of human induced pluripotent stem cells or embryonic stem cells into mesenchymal stem cells, and the induction/differentiation medium comprises: a mesenchymal stem cell basal medium and additives, wherein the additives include: 2 to 10 µM of transforming growth factor-β (TGF-β) inhibitor, 2 to 10 µM of glycogen synthase kinase-3β (GSK3β) inhibitor, and 5 to 10 µM of rho-associated coiled-coil containing protein kinase (ROCK) inhibitor.

2. The induction/differentiation medium according to claim 1, wherein the additives further include: human platelet lysate, ascorbic acid, GlutaMax additive, non-essential amino acids (NEAA), insulin-transferrin-selenium-ethanolamine (ITS-X) supplement, insulin-like growth factor (IGF), basic fibroblast growth factor (bFGF), and platelet-derived growth factor-BB (PDGF-BB); and
preferably, wherein the human platelet lysate is at a volume concentration of 1 to 10%; the ascorbic acid is at a concentration of 30 to 70 mM; the GlutaMax additive is at a volume concentration of 0.5% to 2%; the NEAA is at a volume concentration of 0.5% to 2%; the ITS-X supplement is at a volume concentration of 0.5% to 2%; the IGF is at a concentration of 1 to 10 ng/mL; the bFGF is at a concentration of 1 to 10 ng/mL; and the PDGF-BB is at a concentration of 1 to 10 ng/mL.

3. The induction/differentiation medium according to claim 1, wherein: the TGF-β inhibitor includes at least one of SB431542, A-8301, or LY2157299; the GSK3β inhibitor includes at least one of CHIR99021, SB415286, or LY2090314; and the ROCK inhibitor includes at least one of Y-27632 or Blebbistatin.

4. The induction/differentiation medium according to claim 1, wherein the mesenchymal stem cell basal medium is a serum-free basal medium for mesenchymal stem cells; and the serum-free basal medium for mesenchymal stem cells includes at least one of high-glucose Dulbecco's Modified Eagle Medium (DMEM), Alpha Minimum Essential Medium (Alpha-MEM), or dulbecco's modified eagle medium/ham's f-12 nutrient mixture (DMEM/F12) medium.

5. A method for directly inducing and differentiating human induced pluripotent stem cells or embryonic stem cells into mesenchymal stem cells, the method comprising: subjecting the human induced pluripotent stem cells or the embryonic stem cells to passaging culture in the induction/differentiation medium according to any one of claims 1-4, thereby directly inducing and differentiating the human induced pluripotent stem cells or the embryonic stem cells into the mesenchymal stem cells.

6. The method according to claim 5, comprising:
step I. culturing a cell culture of the human induced pluripotent stem cells or the embryonic stem cells; and
step II. replacing a medium in the cell culture with the induction/differentiation medium to perform passaging culture, thereby obtaining P3~P5 passage mesenchymal stem cells or a cell culture including the P3~P5 passage mesenchymal stem cells.

7. The method according to claim 6, wherein, in step I, the culturing the cell culture includes: culturing the human induced pluripotent stem cells or the embryonic stem cells in a xeno-free medium in a feeder-free culture manner;
preferably, the step of culturing the cell culture further includes: first culturing the human induced pluripotent stem cells or the embryonic stem cells normally in an induced pluripotent stem cell (iPSC) maintenance medium; when confluence of the cell culture reaches 80% to 90%, digesting the human induced pluripotent stem cells with Tryple into a single-cell suspension; resuspending the single-cell suspension in an iPSC maintenance medium; adding the ROCK inhibitor to the iPSC maintenance medium; after maintaining the ROCK inhibitor for 24 h, and replacing with a complete iPSC maintenance medium to obtain the cell culture at 30% to 50% confluence;
preferably, the iPSC maintenance medium includes at least one of Chemically Defined Essential 8 Medium (E8), StemFit Basic04, or mTeSR Plus;
preferably, the ROCK inhibitor includes at least one of Y-27632 or Blebbistatin; a matrix gel used in the culturing the cell culture includes at least one of Laminin-521, Vitronectin, or Matrigel; and
preferably, culturing conditions for the step of culturing the cell culture include: culturing in an incubator at 35-40°C, 4-6% CO₂, and 92-98% humidity.

8. The method according to claim 6, wherein, in step II, replacing the medium in the cell culture with the induction/differentiation medium to perform passaging culture includes: replacing the medium in the cell culture with the induction/differentiation medium; when confluence reaches 80%-90%, designating the cell culture as P0 passage mesenchymal stem cells; performing a digestion treatment on the P0 passage mesenchymal stem cells; resuspending the P0 passage mesenchymal stem cells after the digestion treatment using the induction/differentiation medium; continuing culturing to obtain P1 passage mesenchymal stem cells; and repeating the above passage culturing steps to obtain the P3~P5 passage mesenchymal stem cells; and
preferably, the digestion treatment includes: aspirating supernatant; adding Ca²⁺ and Mg²⁺-free dulbecco's phosphate buffered saline (DPBS) to wash the P0 passage mesenchymal stem cells; performing the digestion treatment on the P0 passage mesenchymal stem cells after washing; centrifuging to remove supernatant; and obtaining the P0 passage mesenchymal stem cells after the digestion treatment.

9. The method according to claim 6, further comprising: placing the P3~P5 passage mesenchymal stem cells or the cell culture including the P3~P5 passage mesenchymal stem cells in an expansion medium for mesenchymal stem cells to perform expansion passaging culture, thereby obtaining the mesenchymal stem cells;
preferably, the expansion medium for mesenchymal stem cells includes: a mesenchymal stem cell basal medium and additives; wherein the additives include: human platelet lysate, ascorbic acid, GlutaMax additive, NEAA, ITS-X supplement, IGF, bFGF, and PDGF-BB;
preferably, the additives further include: human platelet lysate at a volume concentration of 1 to 10%; ascorbic acid at a concentration of 30 to 70 mM; GlutaMax additive at a volume concentration of 0.5% to 2%; NEAA at a volume concentration of 0.5% to 2%; ITS-X supplement at a volume concentration of 0.5% to 2%; IGF at a concentration of 1 to 10 ng/mL; bFGF at a concentration of 1 to 10 ng/mL; and PDGF-BB at a concentration of 1 to 10 ng/mL; and
preferably, the mesenchymal stem cell basal medium is a serum-free basal medium for mesenchymal stem cells; and the serum-free basal medium for mesenchymal stem cells includes at least one of high-glucose DMEM, Alpha-MEM, or DMEM/F12 medium.

10. Mesenchymal stem cells prepared by the method according to any one of claims 5-9,
preferably, the mesenchymal stem cells are multipotent cells capable of differentiating into an adipocyte, an osteocyte, a chondrocyte, a myocyte, a neuron, and a cardiomyocyte;
preferably, the mesenchymal stem cells are capable of expressing cell surface markers Cluster of Differentiation (CD) 29, CD73, CD90, CD105, CD166, and CD44;
preferably, the mesenchymal stem cells do not express cell surface markers Human Leukocyte Antigen-DR isotype (HLA-DR), CD34, CD45, CD19, and CD14; and
preferably, the mesenchymal stem cells are P5 or higher passage mesenchymal stem cells; in the mesenchymal stem cells, a proportion of cells expressing CD29, CD44, CD73, CD90, CD105, and CD166 is not less than 95%; and in the mesenchymal stem cells, a proportion of cells expressing HLA-DR, CD34, CD45, CD19, and CD14 is not more than 2%.
